# EUROPÄISCHE PATENTSCHRIFT

(11) **EP 1 399 476 B1**
(45) Veröffentlichungstag und Bekanntmachung des Hinweises auf die Patenterteilung: **16.08.2006**
(21) Anmeldenummer: 02745379.4
(22) Anmeldetag: 12.06.2002
(51) Int. Cl.: C07K 14/47, G01N 33/569, G01N 33/68

(54) **VERWENDUNG LÖSLICHER CYTOKERATIN-1-FRAGMENTE IN DIAGNOSTIK**
USE OF SOLUBLE CYTOKERATINE-1-FRAGMENTS IN DIAGNOSTICS
UTILISATION DE FRAGMENTS SOLUBLES DE CYTOKERATINE-1 EN DIAGNOSTIC

(30) Priorität: 27.06.2001 DE 10130985
(43) Veröffentlichungstag der Anmeldung: 24.03.2004
(73) Patentinhaber: B.R.A.H.M.S. Aktiengesellschaft, 16761 Hennigsdorf (DE)
(72) Erfinder: BERGMANN, Andreas, 12351 Berlin (DE); STRUCK, Joachim, 12161 Berlin (DE); ÜHLEIN, Monika, 10407 Berlin (DE)
(74) Vertreter: Andrae, Steffen
(86) Internationale Anmeldenummer: PCT/EP2002/006473
(87) Internationale Veröffentlichungsnummer: WO 2003/002600

(56) Entgegenhaltungen:
- WO-A-01/12212
- WO-A-97/25622
- US-A- 5 527 773
- WIEDENMANN B ET AL: "CYTOKERATIN FRAGMENTS IN SERUM POSSIBLE NEW MARKERS FOR THE FOLLOW-UP OF PATIENTS WITH INFLAMMATORY LIVER DISEASE" GASTROENTEROLOGY, Bd. 96, Nr. 5 PART 2, 1989, Seite A673 XP008009392 ISSN: 0016-5085
- KANAZAWA HIROSHI ET AL: "CYFRA 21-1, a cytokeratin subunit 19 fragment, in bronchoalveolar lavage fluid from patients with interstitial lung disease." CLINICAL SCIENCE (LONDON), Bd. 94, Nr. 5, Mai 1998 (1998-05), Seiten 531-535, XP002217297 ISSN: 0143-5221

### SEQUENZPROTOKOLL

<110> B.R.A.H.M.S Diagnostica GmbH
<120> Verwendung löslicher Cytokeratin-1-Fragmente in Diagnostik und Therapie
<130> 3537 AS
<140>
   <141>
<160> 5
<170> PatentIn Ver. 2.1
<210> 1
   <211> 12
   <212> PRT
   <213> Primat (Pavian)
<400> 1
   Ser Leu Asn Asn Gln Phe Ala Ser Phe Ile Asp Lys
     1               5                  10
<210> 2
   <211> 11
   <212> PRT
   <213> Primat (Pavian)
<400> 2
   Thr Asn Ala Glu Asn Glu Phe Val Thr Ile Lys
   1 5 10
<210> 3
   <211> 643
   <212> PRT
   <213> Homo sapiens
<300>
   <302> L.Johnson et al., Structure of a gene for the human epidermal 67-kDa keratin
   <303> Proc. Natl. Acad. Sci. U.S.A.
   <304> 1985
   <305> 82
   <306> 1896-1900
<400> 3
   Ser Arg Gln Phe Ser Ser Arg Ser Gly Tyr Arg Ser Gly Gly Gly Phe
   1 5 10 15
   Ser Ser Gly Ser Ala Gly Ile Ile Asn Tyr Gln Arg Arg Thr Thr Ser
   20 25 30
   Ser Ser Thr Arg Arg Ser Gly Gly Gly Gly Gly Arg Phe Ser Ser Cys
           35                  40                  45
   Gly Gly Gly Gly Gly Ser Phe Gly Ala Gly Gly Gly Phe Gly Ser Arg
       50                  55                  60
   Ser Leu Val Asn Leu Gly Gly Ser Lys Ser Ile Ser Ile Ser Val Ala
   65                  70                  75                  80
   Arg Gly Gly Gly Arg Gly Ser Gly Phe Gly Gly Gly Tyr Gly Gly Gly
                   85                  90                  95
   Gly Phe Gly Gly Gly Gly Phe Gly Gly Gly Gly Phe Gly Gly Gly Gly
               100                 105                 110
   Ile Gly Gly Gly Gly Phe Gly Gly Phe Gly Ser Gly Gly Gly Gly Phe
           115                 120                 125
   Gly Gly Gly Gly Phe Gly Gly Gly Gly Tyr Gly Gly Gly Tyr Gly Pro
       130                 135                 140
   Val Cys Pro Pro Gly Gly Ile Gln Glu Val Thr Ile Asn Gln Ser Leu
   145                 150                 155                 160
   Leu Gln Pro Leu Asn Val Glu Ile Asp Pro Glu Ile Gln Lys Val Lys
                   165                 170                 175
   Ser Arg Glu Arg Glu Gln Ile Lys Ser Leu Asn Asn Gln Phe Ala Ser
               180                 185                 190
   Phe Ile Asp Lys Val Arg Phe Leu Glu Gln Gln Asn Gln Val Leu Gln
           195                 200                 205
   Thr Lys Trp Glu Leu Leu Gln Gln Val Asp Thr Ser Thr Arg Thr His
       210                 215                 220
   Asn Leu Glu Pro Tyr Phe Glu Ser Phe Ile Asn Asn Leu Arg Arg Arg
   225                 230                 235                 240
   Val Asp Gln Leu Lys Ser Asp Gln Ser Arg Leu Asp Ser Glu Leu Lys
                   245                 250                 255
   Asn Met Gln Asp Met Val Glu Asp Tyr Arg Asn Lys Tyr Glu Asp Glu
               260                 265                 270
   Ile Asn Lys Arg Thr Asn Ala Glu Asn Glu Phe Val Thr Ile Lys Lys
           275                 280                 285
   Asp Val Asp Gly Ala Tyr Met Thr Lys Val Asp Leu Gln Ala Lys Leu
       290                 295                 300
   Asp Asn Leu Gln Gln Glu Ile Asp Phe Leu Thr Ala Leu Tyr Gln Ala
   305                 310                 315                 320
   Glu Leu Ser Gln Met Gln Thr Gln Ile Ser Glu Thr Asn Val Ile Leu
                   325                 330                 335
   Ser Met Asp Asn Asn Arg Ser Leu Asp Leu Asp Ser Ile Ile Ala Glu
               340                 345                 350
   Val Lys Ala Gln Asn Glu Asp Ile Ala Gln Lys Ser Lys Ala Trp Ala
           355                 360                 365
   Glu Ser Leu Tyr Gln Ser Lys Tyr Glu Glu Leu Gln Ile Thr Ala Gly
       370                 375                 380
   Arg His Gly Asp Ser Val Arg Asn Ser Lys Ile Glu Ile Ser Glu Leu
   385                 390                 395                 400
   Asn Arg Val Ile Gln Arg Leu Arg Ser Glu Ile Asp Asn Val Lys Lys
                   405                 410                 415
   Gln Ile Ser Asn Leu Gln Gln Ser Ile Ser Asp Ala Glu Gln Arg Gly
               420                 425                 430
   Glu Asn Ala Leu Lys Asp Ala Lys Asn Lys Leu Asn Asp Leu Glu Asp
           435                 440                 445
   Ala Leu Gln Gln Ala Lys Glu Asp Leu Ala Arg Leu Leu Arg Asp Tyr
       450                 455                 460
   Gln Glu Leu Met Asn Thr Lys Leu Ala Leu Asp Leu Glu Ile Ala Thr
   465                 470                 475                 480
   Tyr Arg Thr Leu Leu Glu Gly Glu Glu Ser Arg Met Ser Gly Glu Cys
                   485                 490                 495
   Ala Pro Asn Val Ser Val Ser Val Ser Thr Ser His Thr Thr Ile Ser
               500                 505                 510
   Gly Gly Gly Ser Arg Gly Gly Gly Gly Gly Gly Tyr Gly Ser Gly Gly
           515                 520                 525
   Ser Ser Tyr Gly Ser Gly Gly Gly Ser Tyr Gly Ser Gly Gly Gly Gly
       530                 535                 540
   Gly Gly Gly Arg Gly Ser Tyr Gly Ser Gly Gly Ser Ser Tyr Gly Ser
   545                 550                 555                 560
   Gly Gly Gly Ser Tyr Gly Ser Gly Gly Gly Gly Gly Gly His Gly Ser
                   565                 570                 575
   Tyr Gly Ser Gly Ser Ser Ser Gly Gly Tyr Arg Gly Gly Ser Gly Gly
               580                 585                 590
   Gly Gly Gly Gly Ser Ser Gly Gly Arg Gly Ser Gly Gly Gly Ser Ser
           595                 600                 605
   Gly Gly Ser Ile Gly Gly Arg Gly Ser Ser Ser Gly Gly Val Lys Ser
       610                 615                 620
   Ser Gly Gly Ser Ser Ser Val Arg Phe Val Ser Thr Thr Tyr Ser Gly
   625                 630                 635                 640
   Val Thr Arg
<210> 4
   <211> 9
   <212> PRT
   <213> Homo sapiens
<400> 4
   Asp Val Asp Gly Ala Tyr Met Thr Lys
    1               5
<210> 5
   <211> 113
   <212> PRT
   <213> Homo sapiens
<400> 5
   Ser Leu Asn Asn Gln Phe Ala Ser Phe Ile Asp Lys Val Arg Phe Leu
    1               5                  10                  15
   Glu Gln Gln Asn Gln Val Leu Gln Thr Lys Trp Glu Leu Leu Gln Gln
               20                  25                  30
   Val Asp Thr Ser Thr Arg Thr His Asn Leu Glu Pro Tyr Phe Glu Ser
           35                  40                  45
   Phe Ile Asn Asn Leu Arg Arg Arg Val Asp Gln Leu Lys Ser Asp Gln
       50                  55                  60
   Ser Arg Leu Asp Ser Glu Leu Lys Asn Met Gln Asp Met Val Glu Asp
   65                  70                  75                  80
   Tyr Arg Asn Lys Tyr Glu Asp Glu Ile Asn Lys Arg Thr Asn Ala Glu
                   85                  90                  95
   Asn Glu Phe Val Thr Ile Lys Lys Asp Val Asp Gly Ala Tyr Met Thr
               100                 105                 110
   Lys

## Beschreibung

Die vorliegende Erfindung betrifft die Verwendung neuer löslicher Cytokeratin-1-Fragmente in der medizinischen Diagnostik Sie beruht auf dem erstmaligen Nachweis des physiologischen Auftretens bestimmter Peptide in Form löslicher Cytokeratin-1-Fragmente im Zusammenhang mit einem Krankheitsgeschehen, und zwar bei einer experimentell bei einem Primaten erzeugten Sepsis bzw. systemischen Entzündung. Wenn in der vorliegenden Anmeldung der Begriff "Peptide" verwendet wird, erfolgt die Verwendung im Sinne eines Oberbegriffs, der Kondensationsprodukte von Aminosäuren unabhängig von der Länge der gebildeten Kette umfassen soll, also insbesondere Produkte, die unter Berücksichtigung ihrer Kettenlänge als Oligopeptide, Polypeptide oder Proteine bezeichnet werden können.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird nunmehr Ergebnisse eines anderen, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Diese therapeutischen Ansätze laufen darauf hinaus, die Konzentrationen entzündungsfördernder Substanzen zu senken bzw. die Entstehung derartiger Substanzen zu hemmen, und zwar insbesondere unter Verwendung von spezifischen Antikörpern (gegen TNF-α bzw. MIF; vgl. E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170) bzw. die Konzentration von hemmend in die Entzündungskaskade eingreifenden endogenen Substanzen (Protein C; K.Garber, "Protein C may be sepsis solution", Nature Biotechnology, Vol.18, 2000, 917-918) zu erhöhen. In der,letztgenannten Veröffentlichung findet sich ein Überblick über derartige, leider bisher meist wenig erfolgreiche Versuche, das Entzündungsgeschehen unter Beeinflussung ausgewählter endogener Target-Moleküle therapeutisch zu beeinflussen. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Gemäß der vorliegenden Erfindung werden neue lösliche Peptidfragmente angegeben, die in Primaten und Menschen bei infektiös bedingten Entzündungen gebildet werden und für die Entzündungsdiagnostik/Sepsisdiagnostik geeignet sind.

Die vorliegende Erfindung offenbart lösliche Cytokeratin-1-Fragmente, die sich aufgrund ihres spezifischen Auftretens nach einer künstlichen Sepsisauslösung durch Endotoxinverabreichung an Primaten als sepsisspezifische bzw. entzündungsspezifische humane Peptide erwiesen haben.

Die Verwendungen in der medizinischen Diagnostik, bzw. die diagnostischen Bestimmungsverfahren, die sich aufgrund des erstmals nachgewiesenen Auftretens von bestimmten löslichen Cytokeratin-1-Fragmenten bei Sepsis ergeben, werden in den Ansprüchen 1 bis 4 beansprucht.

Anspruch 5 betrifft Antikörper, die spezifisch an die löslichen Cytokeratinfragmente binden, sowie deren Verwendung in Verfahren gemäß Anspruch 4.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere ein nur bei den behandelten Tieren identifizierbares Peptid- bzw. Proteinprodukt gefunden werden konnte. Dieses spezifische Produkt wurde aus dem Elektrophoresegel isoliert und auf an sich bekannte Weise massenspektrometisch untersucht.

Eine erste Teilsequenz des isolierten, trypsinverdauten Proteinspots aus 12 Aminosäuren mit einer Masse M/Z von 692,39 (SEQ ID NO:1) sowie eine zweite Teilsequenz aus 11 Aminosäuren mit einer Masse M/Z von 633,4 (SEQ ID NO:2) konnten eindeutig identifiziert werden, und durch Vergleich der Sequenzen dieser Teilpeptide mit den Daten einer humanen Datenbank mit bekannten Proteinsequenzen konnten die Sequenzen als Fragmente des bekannten, an sich jedoch völlig unlöslichen Cytoskelett-Proteins Cytokeratin-1 (SEQ ID NO:3; vgl. L.Johnson et al., Structure of a gene for the human epidermal 67-kDa keratin; Proc.Natl.Acad.Sci.U.S.A. 82:1896-1900, (1985); Datenbank NiceProt View of SWISS-PROT: Accession number P04264) identifiziert werden. Die beiden Fragmente gemäß SEQ ID NO:1 und SEQ ID NO:2 entsprechen der Sequenz der Aminosäuren 185-196 bzw. 277-287 des vollständigen Cytokeratins-1. Eine weiteres Fragment des Massenspektrums mit einer Masse M/Z (Z=1) von 999,49 stimmt mit einem Fragment von 9 Aminosäuren (SEQ ID NO:4; errechnete Masse 999,45) überein, das der Teilsequenz der Aminosäuren 289-297 des vollständigen Cytokeratins-1 (SEQ ID NO:3) entspricht.

Diese Ergebnisse erlauben den sicheren Schluss, dass das aus dem Elektrophoresegel isolierte Peptid in Form eines Cytokeratin-1-Fragments die Sequenz der Aminosäuren 185-297 (SEQ ID NO:5) von Cytokeratin-1 umfaßt. Das dieser Sequenz (SEQ ID NO:5) entsprechende Fragment hat jedoch nur eine Molekulargewicht von 13615, während das gelelektrophoretisch bestimmte Molekulargewicht des gefundenen Fragments 15700 ± 500 Dalton betrug. Daher sollen als erfindungsgemäße löslichen Cytokeratin-1-Fragmente insbesondere solche angesehen werden, bei denen das Fragment 185-297 (SEQ ID NO:5) an einem oder beiden seiner Enden um bis zu insgesamt 20 Aminosäuren verlängert ist.

Die Identifizierung eines bestimmten löslichen, bei Sepsis in der Leber gebildeten Fragments von Cytokeratin-1 ist von hohem wissenschaftlichen, diagnostischen und therapeutischen Interesse.

Cytokeratin-1 ist ein Proteine aus der Gruppe der als Cytokeratine oder "weiche" Keratine bezeichneten Strukturproteine (Skleroproteine), die als Bestandteile des Cytoskeletts die sog. intermediären Filamente (IF) ausbilden. Sie verleihen der Zelle Formbeständigkeit und zeichnen sich durch eine hohe mechanische und chemische Beständigkeit aus. Unter üblichen physiologischen Bedingungen sind sie wie alle Keratine gegen Proteasen beständig, und es gibt nur wenige Organismen wie z.B. die Kleidermotte und den Pilz Tritirachium album, die Enzymsysteme aufweisen, die in der Lage sind, Keratine abzubauen und als Nahrungsquelle zu nutzen.

Cytokeratine weisen eine Struktur mit einem α-helikalen zentralen Abschnitt von etwa 310-315 Aminosäuren und daran angrenzenden Endabschnitten auf. Die Cytokeratine lassen sich auf der Grundlage der bei ihnen vorkommenden Aminosäuresequenzen und ihrer Ladung zwei Typen zuordnen. Zu Typ I, der saure Proteine umfasst, gehören die Cytokeratine 9-20, während die Cytokeratine 1-8 Typ II zugeordnet werden, der basische oder neutrale Proteine umfasst. Cytokeratine des Typs I und Typs II treten unter Bildung von Heterodimeren in bestimmten Paarungen auf, die für bestimmte Typen von Epithelzellen und damit auch Gewebe charakteristisch sind. Es kann in diesem Zusammenhang z.B. verwiesen werden auf den Übersichtsartikel von R.B.Presland et al., in: Crit Rev Oral Biol Med, 11(4):383-408 (2000) oder M. Bishr Omary et al., Keratin Modifications and Solubility Properties in Epithelial Cells and in vitro; in: Subcellular Biochemistry, Vol.31: Intermediate Filaments, N.Y. 1998, S.105-150.

Eine Bestimmung von IF-Proteinen, zu denen die Cytokeratine gehören, erfolgt bisher vorwiegend im Zusammenhang mit der Tumordiagnose, da es die Identifizierung von bestimmten IF-Proteinen in Metastasen oder Gewebsläsionen ermöglicht, diese einem Ursprungsgewebe oder Primärtumor zuzuordnen.

Gemäß EP-A-0 163 304 erfolgt eine solche Bestimmung mit geeigneten spezifischen Antikörpern auf histodiagnostischem Wege.

In der EP-B1-0 267 355 wird vorgeschlagen, zur Vereinfachung der Durchführung von Bestimmungen der genannten Art die unlöslichen IF-Proteine einer Behandlung zu unterwerfen, die zu deren Abbau führt, und dann die auf diese Weise künstlich erzeugten löslichen Fragmente zu bestimmen. Es wird ferner davon gesprochen, dass Zell-Läsionen auch von einem proteolytischen Abbau unlöslicher Strukturproteine begleitet sein können, so dass unter bestimmten Umständen auch α-helikale Fragmente in Körperflüssigkeiten gefunden werden können.

EP-B1-0 267 356 ist verwandt, betrifft jedoch nicht die direkte Bestimmung von löslichen Fragmenten von IF-Proteinen, sondern von gegen solche Fragmente gebildeten Antikörpern in Serum.

EP-B1-0 337 057 bildet das Verfahren aus EP-B1-0 267 355 weiter als Verfahren zur Identifizierung des Ursprungs einer Zell- oder Gewebsprobe, wobei man einen bestimmten Standard zur Verwendung bei den Bestimmungen vorschlägt.

Die Bestimmung von löslichen Cytokeratin-19-Fragmenten zur Differentialdiagnose, Prognose, Therapieeffizienzkontrolle und Verlaufsbeobachtung in der Nachsorge von Bronchialkarzinomen wird auch in P. Stieber, CYFRA 21-1 (Cytokeratin-19-Fragmente) in: L.Thomas, Labor und Diagnose, S. 987-992 beschrieben. Die Vielseitigkeit von Cytokeratinen, und zwar wiederum insbesondere der Cytokeratine 8, 18 und 19, als Tumormarker wird auch diskutiert in Torgny Stigbrand, The Versatility of Cyotkeratins as Tumor Markers, Tumor Biol 2001, 22:1-3.

In allen obigen Fällen geht es in erster Linie um die Bestimmung von Fragmenten der Cytokeratine 8, 18 und 19. Unter allen in der Literatur diskutierten löslichen Cytokeratin-Fragmenten finden sich keine löslichen Cytokeratin-1-Fragmente, und es fehlen auch alle Hinweise auf ein mögliches Auftreten derartiger Cytokeratin-1 Fragmente bei bestimmten Krankheitszuständen.

Für Cytokeratin-1 galt lange, dass dieses - zusammen mit Cytokeratin-10 - im wesentlichen nur in einem einzigen, immunologisch isolierten Zelltyp, nämlich in suprabasalen ausdifferenzierten Keratinocyten, gefunden wird.

Ein ungewöhnliches Auftreten von Cytokeratin-1 und eine unerwartete Beteiligung an einem Krankheitsgeschehen wird in den Arbeiten von Steven D. Lucas et al., Identifikation of a 35 kD Tumor-Associated Autoantigen in Papillary Thyroid Carcinoma, Anticancer Research 16:2493-2496 (1996), und Steven D. Lucas et al., Aberrantly Expressed Cytokeratin 1, A Tumor-Associated Autoantigen in Papillary Thyroid Carcinoma, Int.J.Cancer 73, 171-177 (1997) im Zusammenhang mit dem papillären Schilddrüsenkarzinom (PTC) beschrieben. Cytokeratin-1 kann dabei in Form eines 35 kD Fragments in einem Solubilisat von PTC-Zellen durch Immunpräzipitation mit Hilfe von Patientenseren nachgewiesen werden. In den Patientenseren werden (Auto)-Antikörper gegen Cytokeratin-1 gefunden. Von löslichen Cytokeratin-1-Fragmenten aus Körperflüssigkeiten und/oder Körpergeweben ist nicht die Rede.

Der erfindungsgemäße Nachweis eines löslichen Cytokeratin-1-Fragments in der Leber von Primaten, bei denen durch Toxinverabreichung eine künstliche Sepsis ausgelöst wurde, bei gleichzeitigem vollständigen Fehlen eines derartigen Fragments in ansonsten völlig gleich behandelten Proben von Kontrolltieren, ist angesichts aller bisherigen Kenntisse über das Auftreten, die Eigenschaften und die Rolle von Cytokeratin-1 höchst überraschend. Da das Auftreten nur bei den behandelten Tieren zu beobachten war, und zwar bereits relativ kurze Zeit nach der Sepsisauslösung durch Toxinverabreichung, ist es möglich, diese Tatsache zur Schaffung eines vielversprechenden diagnostischen Sepsis-, Infektions- und Entzündungs-Nachweisverfahrens durch Bestimmung dieses vom Organismus gebildeten Fragments zu nutzen.

Da davon auszugehen ist, dass das nachgewiesene Fragment infolge einer erhöhten sepsis- bzw. entzündungsspezifischen Proteaseaktivität (und/oder einer verminderten Inhibierung einer solchen Proteaseaktivität durch die normalerweise an der Regulierung beteiligten Proteaseinhibitoren) auf proteolytischem Wege aus dem vollständigen Cytokeratin-1 gebildet wird, jedoch nicht durch eine modifizierte Expression eines entsprechenden Gens, ist zu erwarten, dass sich auch noch weitere lösliche Cytokeratin-1-Fragmente, die "restlichen" Bereichen des proteolytisch abgebauten vollständigen Cytokeratins-1, insbesondere seines α-helikalen Bereichs, entsprechen, finden lassen und ähnlich wie das o.g. Fragment für eine Bestimmung eignen. Die Bestimmung derartiger weiterer Fragments soll ausdrücklich als Variante von der vorliegenden Erfindung mit umfaßt werden.

Da Cytokeratin-1 in den jeweiliegen Zellen in der Regel gemeinsam mit Cytokeration-10 gefunden wird, erscheint es auch möglich, dass bei der infektions- bzw. entzündungsbedingten erhöhten proteolytischen Aktivität des Organismus unter Läsion von Zellen, die die beiden Cytokeratine 1 und 10 enthalten, auch Cytokeratin-10-Fragmente gebildet werden und wie Cytokeratin-1-Fragmente bestimmt werden können. Die Cytokeratin-1-Fragmente können ggf. auch als lösliche Addukte oder Aggregate vorliegen, z.B. in Form von Aggregaten mit Cytokeratin-10-Fragmenten.

Es liegt ferner im Bereich der vorliegenden Erfindung, Cytokeratin-1-Fragmente zu bestimmen, die im Bereich der Sequenzen SEQ ID NO: 5 gewisse Abweichungen von den beschriebenen konkreten Aminosäure-Teilsequenzen aufweisen, insbesondere dann, wenn im Bereich der genannten Sequenzen individuelle, patientenspezifische Abweichungen oder Polymorphien beobachtet werden können. Die Übereinstimmung mit den genannten konkreten Sequenzen ist dabei jedoch voraussichtlich höher als 75%, und insbesondere auch höher als 90% bzw. 95%.

Die Bestimmungen können dabei nach irgendeinem beliebigen geeigneten Nachweisverfahren erfolgen, wobei jedoch die Bestimmung in einer Körperflüssigkeit eines Patienten auf immundiagnostischem Wege unter Verwendung geeigneter selektiver Antikörper unter praktischen Gesichtspunkten am vorteilhaftesten erscheint.

Aufgrund der Tatsache, dass bei einer experimentellen Sepsisauslösung erstmals ein bestimmtes lösliches Fragment des an sich völlig unlöslichen Cytokeratins-1 nachgewiesen werden konnte, wird somit die Möglichkeit geschaffen, derartige Cytokeratin-1-Fragmente für diagnostische und/oder therapeutische Zwecke zu nutzen. Dafür können Cytokeratin-1-Fragmente ggf. auch nach Verfahren, die inzwischen zum Stand der Technik gehören, synthetisch, gentechnologisch oder auch hydrolytisch, insbesondere proteolytisch, gezielt hergestellt werden.

Ferner können Cytokeratin-1-Fragmente bzw. geeignete Teilsequenzen davon nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung von Cytokeratin-1-Fragmenten in Körperflüssigkeiten eines Patienten und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik und muss nicht besonders beschrieben werden. Ferner ist auch ausdrücklich die Antikörpererzeugung unter Anwendung von Techniken der direkten genetischen Immunisierung mit einer entsprechenden DNA zu erwähnen. Es liegt somit im Rahmen der vorliegenden Erfindung, zur Immunisierung z.B. eine cDNA von Cytokeratin-1-Fragmenten zu - verwenden, da es sich in der Vergangenheit gezeigt hat, dass bei der Anwendung derartiger Immunisierungstechniken das Spektrum der gewinnbaren Antikörper erweitert werden kann. Es können aber auch bereits bekannte und im Handel erhältliche Antikörper gegen Cytokeratine verwendet werden.

Bei der immunologischen Bestimmung von löslichen cytokeratin-1-Fragmenten kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen von Cytokeratin-1-Fragmenten darstellen. Variationen der beschriebenen Techniken und/oder weitere Immunisierungstechniken kann der Fachmann einschlägigen Standardwerken und Veröffentlichungen entnehmen und sinngemäß anwenden.

Cytokeratin-1-Fragmente mit den Teilsequenzen gemäß SEQ ID NO:1, SEQ ID NO: 2, SEQ ID NO:4 und/oder SEQ ID NO: 5' oder Teilpeptide davon, oder Cytokeratin-1-Fragmente, die bei der proteolytischen Spaltung des α-helikalen Teils von Cytokeratin-1 neben dem obigen nachgewiesenen und charakterisierten Fragment gebildet werden, können aufgrund der vorliegenden Ergebnisse als spezifische Markerpeptide (Biomarker) zum diagnostischen Nachweis, zur Verlaufsprognose und zur Verlaufskontrolle von Entzündungen und Infektionen (insbesondere auch von systemischen Infektionen vom Sepsistyp) dienen. Wie die Bestimmung von Procalcitonin kann dabei die Bestimmung mindestens des konkreten gefundenen löslichen Cytokeratin-1-Fragments zur differentialdiagnostischen Früherkennung und zur Erkennung sowie zur Verlaufsprognose, zur Beurteilung des Schweregrads und zur therapiebegleitenden Verlaufsbeurteilung von Sepsis und Infektionen erfolgen, wobei man bei einem derartigen Verfahren in einer Probe einer biologischen Flüssigkeit oder eines Gewebes eines Patienten den Gehalt eines bestimmten Cytokeratin-1-Fragments bestimmt und aus der festgestellten Anwesenheit und/oder Menge des bestimmten Peptids auf das Vorliegen einer Entzündung, einer schweren Infektion oder einer Sepsis schließt und das erhaltene Ergebnis mit dem Schweregrad der Sepsis korreliert und die Behandlungsmöglichkeiten und/oder die Behandlungsaussichten abschätzt.

Cytokeratin-1-Fragmente (oder ggf. für solche kodierende DNA-Abschnitte) können jedoch auch in der präventiven Medizin oder Therapie verwendet werden.

Für eine therapeutische Nutzung der neuen erfindungsgemäßen Erkenntnisse spielt eine besondere Rolle, dass in jüngster Zeit gefunden wurde, dass Cytokeratin-1 eine wichtige Rolle als eine Art Oberflächenrezeptor für das sog. hochmolekulare Kinin (HK) spielt, dessen Bindung ein wichtiger Schritt bei der Auslösung der Bradykinin-Kaskade ist. Es wird in diesem Zusammenhang verwiesen auf die in den folgenden Arbeiten zu findenden Ergebnisse: (a) Zia Shariat-Madar et al., Kininogen-Cytokeratin 1 Interactions in Endothelial Cell Biology; TCM Vol.9, No.8, 1999, 238-244; (b) Zia Shariat-Madar et al., Mapping Binding Domains of Kininogens on Endothelial Cell Cytokeratin 1; J.Biol.Chem. 273, No.11, 7137-7145, 1999; (c) K. Joseph et al., Factor XII-dependent Contact Actvation on Endothelial Cells and Binding proteins gClqR and Cytokeratin 1, Thromb.Haemost. 85:119-124, 2001; (d) K.Joseph et al., Activation of the Kinin-Forming Cascade on the Surface of Endothelial Cells, Biol.Chem. Vol.382:71-75 (2001); (e) A.Kaplan et al., Activation of the Plasma Kinin Forming Cascade along Cell Surfaces, Int Arch Allergy Immunol 2001; 124:339-342.

Als Bindungsstelle für das genannte HK an Cytokeratin-1 wurde in der o.g. Veröffentlichung (b) ein Sequenzbereich ermittelt, der im unmittelbaren Randbereich des experimentell nachgewiesenen Cytokeratin-1-Fragments mit der Sequenz SEQ ID NO:5 liegt.

Die Kinine der sog. Bradykinin-Kaskade haben u.a. vasodilatatorische, blutdrucksenkende und die Gefäßpermeabilität erhöhende Wirkungen. Da bei einer Sepsis derartige Symptome beobachtet werden und zu den für den Patienten kritischen physiologischen Vorgängen zählen, ist es nicht ausgeschlossen, dass ein ursächlicher Zusammenhang zwischen den genannten klinischen Symptomen und einem durch die Sepsis ausgelösten plötzlichen Auftreten von löslichen Cytokeratin-1-Fragmenten im Blut, verbunden mit einer direkten Beeinflussung der Bradykinin-Kaskade, besteht. Es ist sowohl eine verstärkende Wirkung, im Sinne eines erhöhten, nicht mehr lokal beschränkten Angebots an aktivierenden HK-Rezeptoren, als auch eine Beeinflussung im Sinne einer Gegenregulation, z.B. durch Bindung und Inaktivierung von im Übermaß ausgeschütteten Kininen, denkbar, und es ist auch denkbar, dass derartige Wirkungen in einer konzentrationsabhängigen Weise zeitlich aufeinander folgen.

Das macht Cytokeratin-1-Fragmente auch zu potentiellen vielversprechenden therapeutischen Targets für die Therapie von Sepsis und ähnlichen schweren Entzündungen. In diesem Zusammenhang kann man je nachdem, welche der o.g. Wirkungen sich als die wichtigere herausstellt, entweder lösliche Cytokeratin-1-Fragmente in Form von Arzneimitteln an einen septischen oder sepsisgefährdeten Patienten verabreichen, oder man kann die Konzentration derartiger Fragmente durch Verabreichung von bindenden Antikörpern, oder durch extrakorporale Entfernung derartiger Fragmente im Sinne einer Blutwäsche oder Plasmapherese mittels Affinitätsabsorption, vermindern. Die Kinin-Kaskade kann dadurch beeinflusst werden, was sich als lebensrettend erweisen kann.

Wenn man lösliche Cytokeratin-1 Fragmente als solche für therapeutische Zwecke in Arzneimitteln einsetzt, kann man sie auch durch gezielte Proteolyse mit geeigneten Endoproteasen künstlich herstellen, und zwar unter Einsatz von Cytokeratin-1-Isolaten oder -Konzentraten, die man unter Ausnutzung der bekannten Löslichkeitseigenschaften der unterschiedlichen Cytokeratine in Puffern aus leicht zugänglichen Materialien herstellen kann. Verwendet man als Ausgangsmaterial körpereigenes Material eines Patienten (autologes oder autogenisches Material), ist eine optimale Verträglichkeit und Wirksamkeit gewährleistet.

Als therapeutisch verwendbare Cytokeratin-1-Fragmente sollen dabei auch solche Moleküle anzusehen sein, die das gewählte Cytokeratin-1-Fragment in posttranslational modifizierter Form, z.B. in glykosylierter oder phosphorylierter Form, oder auch in einer durch pharmazeutische Hilfsstoffe, z.B. Polyethylenglykolreste, substituierten Form enthalten.

Nachfolgend wird die Auffindung und Identifizierung eines spezifischen Cytokeratin-1-Fragments in näheren Einzelheiten geschildert, wobei auf das beigefügte Sequenzprotokoll bezug genommen wird. Die Figuren zeigen:
- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Position des erfindungsgemäßen sepsisspezifischen Produkts (Cytokeratin-1-Fragments) an, das in Darstellung (B) durch einen Kreis hervorgehoben ist;

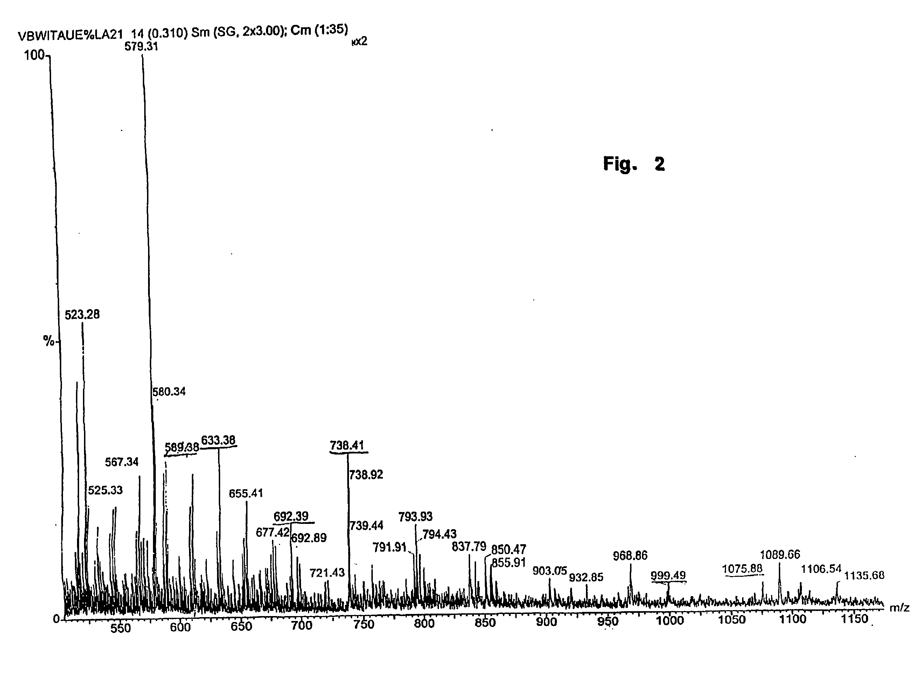

- Fig. 2: das Massenspektrum des durch 2D-Gelelektrophorese identifizierten trypsinverdauten isolierten Produkts, und
- Fig. 3a: die Ergebnisse einer Tandem-Elektrophorese eines selektierten Peptidfragments der Trypsinverdauung mit einem Ladung/Masse-Verhältnis von 692,39; und
- Fig. 3b: die Ergebnisse einer Tandem-Elektrophorese, eines weiteren selektierten Peptidfragments der Trypsinverdauung mit einem Ladung/Masse-Verhältnis von 633,38.
- Fig.4: die Ergebnisse der Bestimmung des löslichen Cytokeratin-1-Fragments in den Seren von 20 Sepsispatienten im Vergleich mit einer Gruppe von 16 Kontrollpersonen (Blutspendern).

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 gg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Einfrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Unter Verwendung der auf diese Weise gewonnenen Gewebeextrakte wurde zuerst untersucht, in welchem der untersuchten Gewebe sich durch die Endotoxinverabreichung die höchsten Mengen des bekannten Sepsis-Biomarkers Procalcitonin erzeugen lassen. In dem ermittelten Gewebe mit der höchsten Procalcitoninbildung wurde dann mittels differentieller Proteomanalyse nach weiteren bisher nicht identifizierten proteinischen Produkten gesucht, die nur nach der Endotoxinverabreichung auftraten. Dazu wurden als Kontroll-Gewebeproben Gewebeproben unbehandelter Paviane verwendet, wobei die Tötung und Probengewinnung unter identischen Bedingungen erfolgte wie bei den behandelten Tieren.

### 2. Ermittlung von Paviangeweben mit der höchsten Procalcitoninbildung nach Endotoxininjektion.

Proben der einzelnen Gewebe wurden mit Hilfe eines Immunoluminometrischen Tests untersucht, der (in Anlehnung an den zur Bestimmung von humanem Procalcitonin entwickelten LU-MItest® PCT der Anmelderin) mit einem auf Polystyrolröhrchen immobilisierten Antikörper gegen Pavian-Calcitonin einerseits und einem mit einem Akridiniumester markierten monoklonalen Antikörper, der gegen den N-Terminus des Pavian-Procalcitonins gerichtet ist, arbeitet. Mit Hilfe dieses Tests wurden die Gehalte an Pavian-Procalcitonin in den einzelnen Proben nach Kalibrierung des Tests unter Verwendung von rekömbinantem humanem Procalcitonin ermittelt.

Die Versuche ergaben, dass Lebergewebe die größte Procalcitoninmenge liefert. Für die Suche nach neuen sepsisspezifischen Biomarkern wurde daher mit den auf die eingangs beschriebene Weise gewonnenen Proteinextrakten aus Pavianleber gearbeitet.

### 3. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

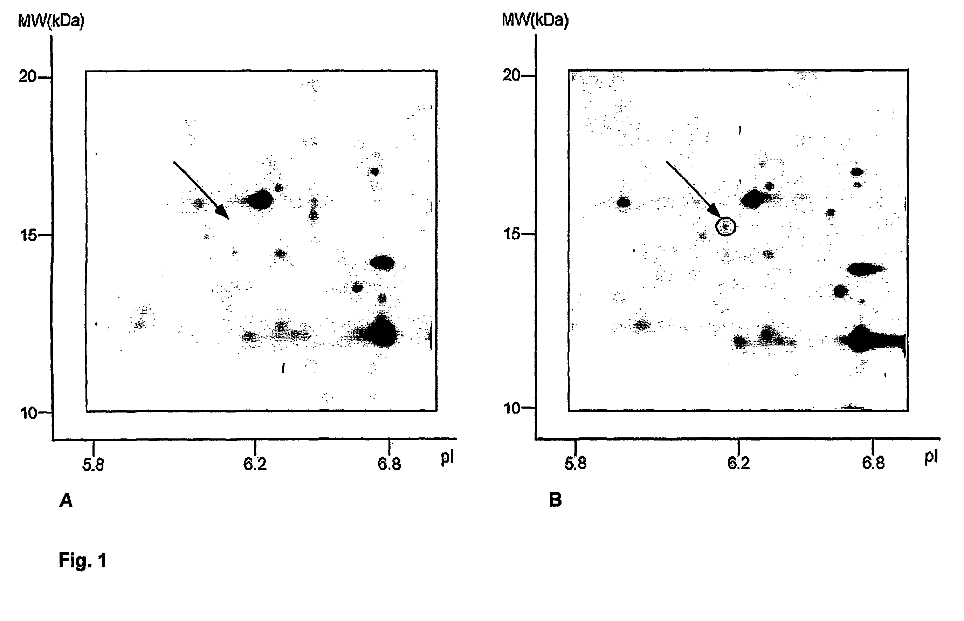

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) dem neuen löslichen Cytokeratin-1-Fragment entspricht, dessen Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Die für die weitere Analyse vorselektierten Proteinspots wurden aus dem Gel ausgeschnitten, unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Dabei wurden die trypsinverdauten Proben nach einer ESI (ElectroSprayIonisierung) einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 4. Identifizierung von eines löslichen Cytokeratin-1-Fragments

Wie in den Figuren 1(A) und 1(B) gezeigt ist, findet sich in Leberzellextrakten von Pavianen, denen eine LPS-Injektion verabreicht worden war, u.a. ein neues Protein, für das aufgrund der Gelektrophoresedaten im Vergleich mit Markersubstanzen mit bekanntem Molekulargewicht ein Molekulargewicht von ca. 15700 ± 500 Dalton abgeschätzt wurde, während aus der relativen Position des Proteins aus der ersten Dimension ein isoelektrischer Punkt von ca. 5,5 bis 6,5 abgeschätzt wurde.

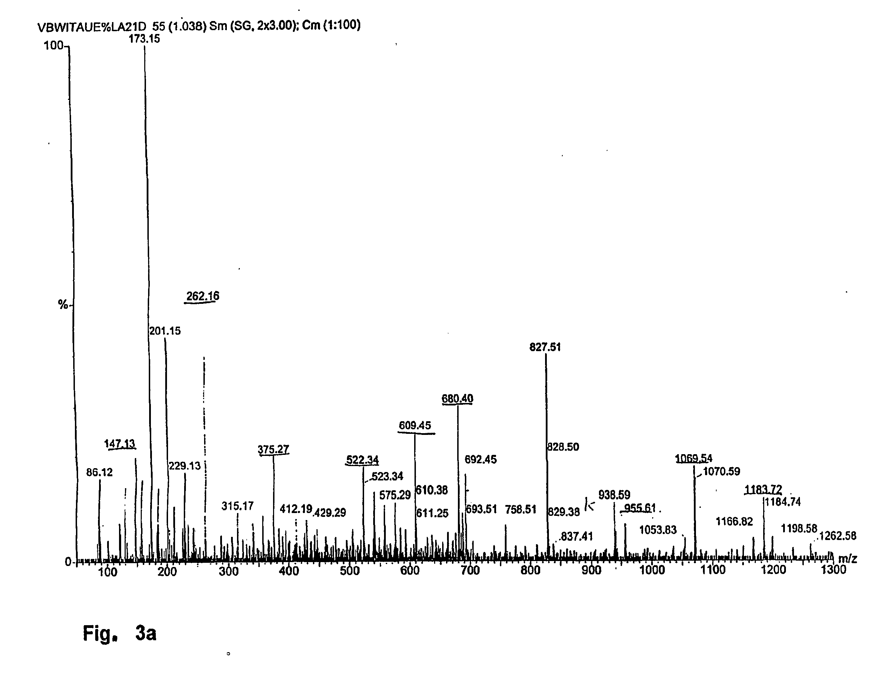

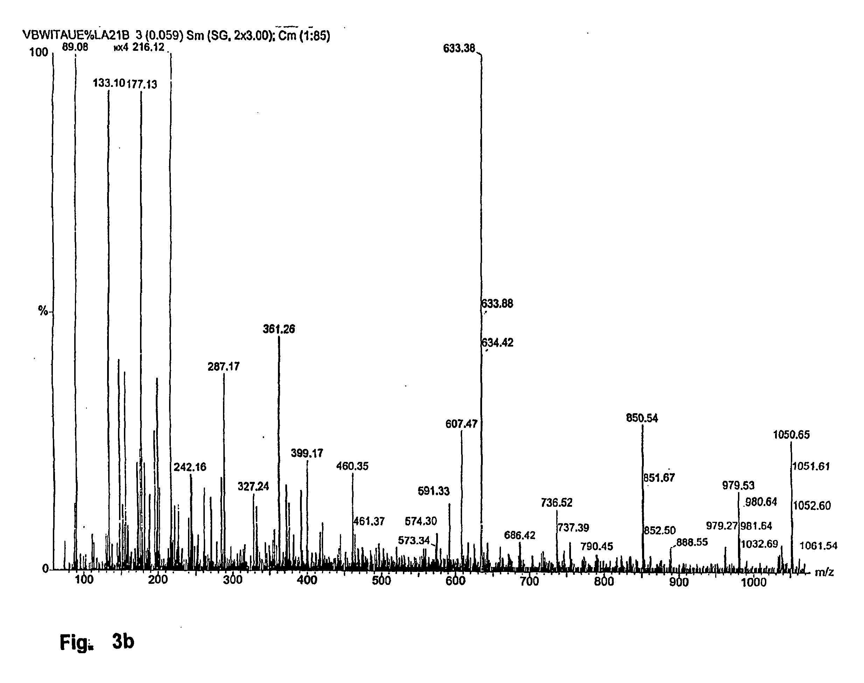

Dieses Protein wurde wie oben massenspektrometrisch analysiert, und den beiden Trypsinfragmente gemäß den Figuren 3a und 3b konnten die Aminosäuresequenzen SEQ ID NO:1 und SEQ ID NO:2 zugeordnet werden, die sich als Teilsequenzen der bekannten Sequenz von Cytokeratin-1 erwiesen (SEQ ID NO:3; vgl. L.Johnson et al., Structure of a gene for the human epidermal 67-kDa keratin; Proc.Natl.Acad.Sci.U.S.A.; 82:1896-1900, (1985); Datenbank NiceProt View of SWISS-PROT: Accession number P04264). Die beiden Fragmente gemäß SEQ ID NO:1 und SEQ ID NO:2 entsprechen der Sequenz der Aminosäuren 185-196 bzw. 277-287 des vollständigen Cytokeratins-1. Eine weiteres Fragment des Massenspektrums mit einer Masse M/Z (Z=1) von 999,49 stimmt mit einem Fragment von 9 Aminosäuren (SEQ ID NO:4; errechnete Masse 999,45) überein, das der Teilsequenz der Aminosäuren 289-297 des vollständigen Cytokeratins-1 (SEQ ID NO:3) entspricht.

Diese Ergebnisse erlauben den sicheren Schluss, dass das aus dem Elektrophoresegel isolierte Peptid in Form eines Cytokeratin-1-Fragments die Sequenz der Aminosäuren 185-297 (SEQ ID NO:5) von Cytokeratin-1 umfaßt. Das dieser Sequenz (SEQ ID NO:5) entsprechende Fragment hat jedoch nur eine Molekulargewicht von 13615, während das gelelektrophoretisch bestimmte Molekulargewicht des gefundenen Fragments 15700 ± 500 Dalton betrug. Daher sollen als erfindungsgemäße löslichen Cytokeratin-1-Fragmente insbesondere solche angesehen werden, bei denen das Fragment 185-297 (SEQ ID NO:5) an einem oder beiden seiner Enden um bis zu insgesamt 20 Aminosäuren verlängert ist.

### 5. Bestimmung des löslichen Cytokeratin-1-Fragments in Seren

In 20 Seren von Sepsis-Patienten, bei denen hohe Werte für den Sepsismarker Procalcitonin (PCT) gefunden worden waren, wurden die Serumkonzentrationen des o.g. löslichen Cytokeratin-1-fragments bestimmt. Bei 95 % der Sepsisseren wurden stark erhöhte Konzentrationen (mehr als 3 ng/ml) gefunden.

Für die orientierenden Bestimmungen in Sepsisseren wurde ein speziell dafür entwickelter kompetitiver Lumineszenz-Immunoassay eingesetzt, bei dem Schaf-Antikörper gegen ein Peptid, das eine Teilsequenz des Cytokeratin-1-Fragments, die die Aminosäuren 214 bis 229 der SEQ ID NO:3 umfasste, verwendet wurden. Das zur Antikörpergewinnung und als Kompetitor verwendete synthetische Peptid ist unter der Bezeichnung Peptid PLY17 kommerziell erhältlich (Jerini Bio-Tools GmbH).

Zur Durchführung der Bestimmungen wurde wie folgt vorgegangen:
Poylstyrol-Röhrchen (Fa. Greiner) wurden mit 100 ng Peptid (PLY17; SEQ ID NO:1) in 300 µl PBS beschichtet. Nach einer 20-stündigen Inkubation bei Raumtemperatur wurde mit 2 x 4 ml PBS, enthaltend 1% BSA, gewaschen. Die peptidbeschichteten Röhrchen wurden dann als Festphase für die Durchführung der nachfolgenden Messungen eingesetzt, bei denen die immobilisierten Peptide und die Cytokeratin-1-Fragmente aus der Probe um einen in Form eines Antiserums zugesetzten Schaf-Antikörpers gegen die o.g. Teilpeptidsequenz konkurrierten.

Zur Messung wurde nach dem folgenden Schema vorgegangen:
1. Pipettiere in die o.g. Röhrchen 100 µg der Probe (Sepsisserum oder Kontrollserum bzw. Kalibratorlösung);
2. Pipettiere 200 µl Antiserum (1:5000 mit PBS verdünnt);
3. Inkubiere 2 h bei Raumtemperatur unter Schütteln;
4. Wasche den ungebundenen Antikörper aus dem Röhrchen (4 x mit 1 ml PBS befüllt und dekantiert) ;
5. Gebe zur Markierung der festphasen-gebundenen Antikörper einen mit einem Akridiniumester markierten Eselanti-Schaf-Antikörper (B.R.A.H.M.S Diagnostica) in 300 ml PBS, 1 %BSA zu;
6. Entferne nach 2 h Inkubation bei Raumtemperatur den ungebundenen Markierungsantikörper, wasche wie unter 4.;
7. Vermesse den an die Festphase gebundenen Akridiniumester auf bekannte Weise mittels eines Luminometers (Fa. Berthold).

Zur Erstellung eines Eichkurve wurden Lösungen mit bekannten Mengen des o.g. synthetischen Peptids eingesetzt, und die Konzentrationen des löslichen Cytokeratin-1-Fragments wurden durch Vergleich der Messwerte für die Sepsisseren mit der Eichkurve bestimmt.

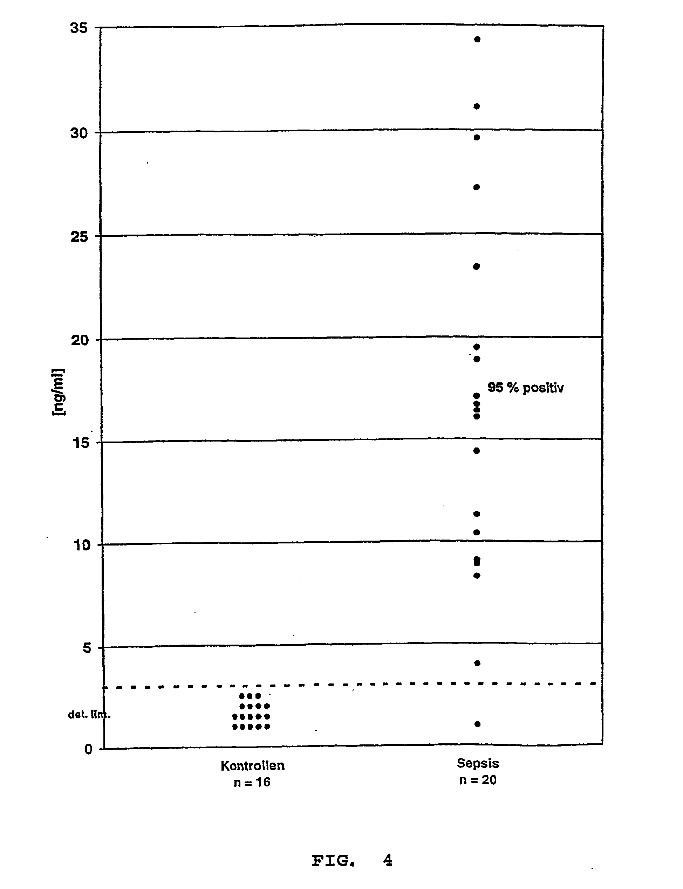

Eine graphische Darstellung der Messergebnisse ist in Figur 4 gezeigt. Bereits mit dem beschriebenen provisorischen, recht einfachen und unempfindlichen kompetitiven Messverfahren wird eine sehr gute Sensitivität der Bestimmung der Cytokeratin-1-Fragmente bei Sepsis ersichtlich.

Das gefundene Cytokeratin-1-Fragment ist als neues Protein (bzw. Peptid) zu bezeichnen, dessen vermutlich proteolytische Bildung erstmals nach Kontakt des Primatenorganismus mit den verabreichten Endotoxinen beobachtet wurde. Über eine mögliche natürliche Rolle des Fragments kann bisher nur spekuliert werden. Seine erstmalige Identifizierung und die dokumentierte hohe Spezifität machen es jedoch zu einem vielversprechenden diagnostischen Target sowie einem neuen interessanten Target für therapeutische Intervention.

Es liegt ferner im Rahmen der vorliegenden Erfindung, das identifizierte Cytokeratin-1-Fragment oder ein verwandtes Fragment, ggf. auch ein Teilfragment, als pharmazeutische Wirkstoffe zu verwenden. Die Erfindung umfasst folglich auch pharmazeutische Zusammensetzungen, die als eigentlichen Wirkstoff eines der erfindungsgemäßen Peptide oder gegen diese Peptide erzeugte, für eine Verabreichung an Patienten aufbereitete Antikörper zusammen mit einem geeigneten pharmazeutischen Träger enthalten.

## Patentansprüche

1. Verwendung von löslichen Cytokeratin-1-Fragmenten aus Körperflüssigkeiten oder Körpergeweben, die die Sequenz der Aminosäuren 185-297 (SEQ ID NO:5) der vollständigen Aminosäuresequenz des Cytokeratins-1 (SEQ ID NO:3) sowie bis zu 20 daran angrenzende Aminosäuren der vollständigen Aminosäuresequenz des Cytokeratins-1 aufweisen und deren gelelektrophöretisch bestimmtes Molekulargewicht 15700 ± 500 Dalton beträgt, oder die eine wenigstens 75%ige, vorzugsweise wenigstens 90%ige Identität mit der Aminosäuresequenz des genannten Fragments aufweisen, als Markerpeptide zum *in vitro* diagnostischen Nachweis, für *in vitro* die Verlaufsprognose und die Verlaufskontrolle von Entzündungen und Infektionen.

2. Verwendung von löslichen Cytokeratin-1-Fragmenten nach Anspruch 1 im Rahmen der differentialdiagnostischen Früherkennung und Erkennung für die Verlaufsprognose, die Beurteilung des Schweregrads und die therapiebegleitende Verlaufsbeurteilung von Sepsis und schweren Infektionen, insbesondere sepsisähnlichen systemischen Infektionen,durch *in vitro* Bestimmung eines ausgewählten löslichen Cytokeratin-1-Fragments in einer biologischen Flüssigkeit oder einer Gewebeprobe eines Patienten.

3. Verfahren zur differentialdiagnostischen Früherkennung und Erkennung, zur Erstellung einer Verlaufsprognose, zur Beurteilung des Schweregrads und zur therapiebegleitenden Verlaufsbeurteilung von Sepsis und schweren Infektionen, insbesondere sepsisähnlichen systemischen Infektionen, **dadurch gekennzeichnet, dass** man die Anwesenheit und/oder Menge eines löslichen Cytokeratin-1-Fragments, das die Sequenz der Aminosäuren 185-297 (SEQ ID NO:5) der vollständigen Aminosäuresequenz des Cytokeratins-1 (SEQ ID NO:3) sowie bis zu 20 daran angrenzende Aminosäuren der vollständigen Aminosäuresequenz des Cytokeratins-1 aufweist und dessen geleektrophoretisch bestimmtes Molekulargewicht 15700 ± 500 Dalton beträgt, in einer biologischen Flüssigkeit oder einer Gewebeprobe eines Patienten *in vitro* bestimmt und aus der Anwesenheit und/oder Menge des bestimmten Fragments Schlüsse hinsichtlich des Vorliegens, des Schweregrads oder des zu erwartenden Verlauf der Sepsis oder Infektion zieht.

4. Verfahren nach Anspruch 3, **dadurch gekennzeichnet, dass** es ein immundiagnostisches Bestimmungsverfahren ist.

5. Antikörper, die spezifisch an lösliche Cytokeratin-1-Fragmente binden, die die Sequenz der Aminosäuren 185-297 (SEQ ID NO:5) der vollständigen Aminosäuresequenz des Cytokeratins-1 (SEQ ID NO:3) sowie bis zu 20 daran angrenzende Aminosäuren der vollständigen Aminosäuresequenz des Cytokeratins-1 aufweisen und deren geleektrophoretisch bestimmtes Molekulargewicht 15700 ± 500 Dalton beträgt.

6. Verwendung von Antikörpern gemäß Anspruch 5 als spezifische Bindungsreagenzien in *in vitro* immundiagnostischen Bestimmungsverfahren gemäß Anspruch 4.

## Claims

1. Use of soluble cytokeratin 1 fragments from body fluids or body tissues comprising the sequence of the amino acids 185-297 (SEQ ID NO:5) of the complete amino acid sequence of cytokeratin 1 (SEQ ID NO:3) and up to 20 adjacent. amino acids of the complete amino acid sequence of cytokeratin 1 and having a molecular weight, determined by gel electrophoresis, of 15,700 ± 500 Dalton, or having at least 75%, preferably at least 90%, identity with the amino acid sequence of said fragment, as marker peptides for the diagnostic in vitro determination, *in vitro* prognosis and monitoring of the course of inflammations and infections.

2. Use of soluble cytokeratin 1 fragments according to Claim 1 in differential early diagnosis and diagnosis for the prognosis of the course, the assessment of the severity and the therapy-accompanying assessment of the course of sepsis and severe infections, in particular sepsis-like systemic infections, by in vitro determination of a selected soluble cytokeratin 1 fragment in a biological fluid or a tissue sample of a patient.

3. Method for the differential early diagnosis and diagnosis, for the preparation of a prognosis of the course, for the assessment of the severity and for the therapy-accompanying assessment of the course of sepsis and severe infections, in particular sepsis-like systemic infections, **characterized in that** the presence and/or amount of a soluble cytokeratin 1 fragment comprising the sequence of the amino acids 185-297 (SEQ ID NO:5) of the complete amino acid sequence of cytokeratin 1 (SEQ ID NO:3) and up to 20 adjacent amino acids of the complete amino acid sequence of cytokeratin 1 and having a molecular weight, determined by gel electrophoresis, of 15,700 ± 500 Dalton is determined *in vitro* in a biological fluid or a tissue sample of a patient and conclusions about the presence, the severity or the expected course of the sepsis or infection are drawn from the presence and/or the amount of the fragment determined.

4. Method according to Claim 3, **characterized in that** it is an immunodiagnostic method of determination.

5. Antibodies specifically binding to soluble cytokeratin 1 fragments comprising the sequence of the amino acids 185-297 (SEQ ID NO:5) of the complete amino acid sequence of cytokeratin 1 (SEQ ID NO:3) and up to 20 adjacent amino acids of the complete amino acid sequence of cytokeratin 1 and having a molecular weight, determined by gel electrophoresis, of 15,700 ± 500 Dalton.

6. Use of antibodies accoding to claim 5 als specific binding reagents in immunodiagnostic *in vitro* methods of determination according to claim 4.

## Revendications

1. Utilisation de fragments de cytokératine-1 solubles provenant de fluides corporels ou de tissus corporels, qui présentent la séquence des acides aminés 185-297 (SEQ ID NO: 5) de la séquence d'acides aminés complète de la cytokératine-1 (SEQ ID NO: 3), de même que jusqu'à 20 acides aminés adjacents de la séquence d'acides aminés complète de la cytokératine-1, et dont le poids moléculaire déterminé par électrophorèse sur gel fait 15700 ± 500 Dalton, ou qui présentent une homologie d'au moins 75%, mieux d'au moins 90% avec la séquence d'acides aminés du fragment cité, comme peptide marqueur pour le diagnostic de détection in vitro, pour le pronostic de l'évolution et le contrôle de l'évolution in vitro d'inflammations et d'infections.

2. Utilisation de fragments de cytokératine-1 solubles selon la revendication 1, dans le cadre du dépistage précoce et du dépistage par diagnostic différentiel pour le pronostic de l'évolution, l'évaluation de la gravité et l'évaluation de l'évolution accompagnant la thérapie de septicémies et d'infections sévères, en particulier d'infections systémiques analogues à une septicémie, par détermination in vitro d'un fragment de cytokératine-1 soluble, choisi, dans un fluide biologique ou un échantillon de tissu d'un patient.

3. Procédé pour le dépistage précoce et le dépistage par diagnostic différentiel, pour l'établissement d'un pronostic d'évolution, pour l'évaluation du degré de gravité et pour l'évaluation de l'évolution accompagnant la thérapie de septicémies et d'infections sévères, en particulier des infections systémiques analogues à une septicémie, **caractérisé en ce que** l'on détermine in vitro, dans un fluide biologique ou un échantillon de tissu d'un patient, la présence et/ou la quantité d'un fragment de cytokératine-1 soluble qui présente la séquence d'acides aminés 185-297 (SEQ ID NO: 5) de la séquence d'acides aminés complète de la cytokératine-1 (SEQ ID NO: 3), de même que jusqu'à 20 acides aminés adjacents de la séquence d'acides aminés complète de la cytokératine-1, et dont le poids moléculaire déterminé par électrophorèse sur gel fait 15700 ± 500 Dalton, et l'on déduit de la présence et/ou de la quantité du fragment déterminé des conséquences concernant l'existence, la gravité ou l'évolution attendue de la septicémie ou de l'infection.

4. Procédé selon la revendication 3, **caractérisé en ce qu'**il s'agit d'un procédé de détermination par diagnostic immunologique.

5. Anticorps qui se lient de manière spécifique aux fragments de cytokératine-1 solubles, qui présentent la séquence d'acides aminés 185-297 (SEQ ID NO: 5) de la séquence d'acides aminés complète de la cytokératine-1 (SEQ ID NO: 3), de même que jusqu'à 20 acides aminés adjacents de la séquence d'acides aminés complète de la cytokératine-1, et dont le poids moléculaire déterminé par électrophorèse sur gel fait 15700 ± 500 Dalton.

6. Utilisation d'anticorps selon la revendication 5, comme réactifs de liaison spécifiques dans le procédé de détermination par diagnostic immunologique in vitro selon la revendication 4.
